Europäisches Patentamt

European Patent Office

Office européen des brevets

(19)

(11) Publication number: **0 333 517**
**A2**

(12) **EUROPEAN PATENT APPLICATION**

(21) Application number: **89302702.9**

(22) Date of filing: **20.03.89**

(51) Int. Cl.⁴: **C 07 K 7/00**
**A 61 K 37/02**

(30) Priority: **18.03.88 US 170171**

(43) Date of publication of application:
**20.09.89 Bulletin 89/38**

(84) Designated Contracting States:
**AT BE CH DE ES FR GB GR IT LI LU NL SE**

(71) Applicant: **THE ROCKEFELLER UNIVERSITY**
**1230 York Avenue**
**New York New York 10021-6399 (US)**

(72) Inventor: **Wright, Samuel D.**
**1161 York Avenue**
**New York New York 10021 (US)**

(74) Representative: **Denmark, James**
**c/o Bailey, Walsh & Co. 5 York Place**
**Leeds LS1 2SD Yorkshire (GB)**

(54) Method and agent for inhibiting the binding of human polymorphonuclear leukocytes to endothelium and compositions therefor.

(57) A method and agent including a pharmaceutical composition for inhibiting the binding of human polymorphonuclear leukocytes (PMN) to endothelium and to fibrinogen has been developed. The administration of certain inhibitory peptides significantly inhibits binding of PMN both to cultured human endothelial cells and to immobilized fibrinogen so that these peptides are useful for inhibiting the binding and/or ingress of PMN at sites of clotting, infection, tissue injury, or inflammation. Infiltration by PMN is a principal cause of tissue destruction in stroke, heart disease, meningitis, pneumococcal pneumonia, otitis media, adult respiratory distress syndrome, gout, asthma, psoriasis, Reiter's syndrome, leukoplastic vasculitis, Sweet's syndrome, pustular vasculitis, erythema nodosum, pyoderma gangrenosum, ostructire trauma, acute rejection of transplanted organs, emphysema and septic shock of all forms so that the peptides of this can be used in treatment of any or all of these diseases.

**Description**

## METHOD AND AGENT FOR INHIBITING THE BINDING OF HUMAN POLYMORPHONUCLEAR LEUKOCYTES TO ENDOTHELIUM AND COMPOSITIONS THEREFOR

### BACKGROUND OF THE INVENTION

Polymorphonuclear (PMN) leukocytes circulate in the blood and do not adhere to the endothelium (the cells lining the vessels). Upon the introduction of a microbe or another inflammatory substance into nearby tissues, PMN are induced to bind to the endothelium and then migrate into the tissues. Since PMN can recognise and kill many microbes, this is a protective mechanism. However, in many disease circumstances, PMN react in an exaggerated and deleterious fashion. They may bind so avidly to endothelium as to occlude blood flow. Once in the tissues, they secrete proteases, reactive oxygen intermediates, and other toxic molecules which not only kill microbes but also can result in extensive tissue damage. In addition, they release inflammatory mediators that alter vascular tone and permeability, and that recruit additional phagocytes to the site, thus perpetuating inflammation.

A molecule on the surface of PMN that mediates adhesion to endothelium has recently been defined. That molecule, CD11b/CD18, is thought to mediate adhesion because (a) monoclonal antibodies against CD11b/CD18 inhibit binding of PMN to endothelium in vitro, (b) injection of monoclonal antibodies against CD11b/CD18 into rabbits prevents normal emigration of PMN in response to an inflammatory stimulus, (c) PMN from patients with a genetic defect in the expression of CD11b/CD18 and the related molecules, CD11a/CD18 and CD11c/CD18, fail to bind to endothelial cells in vitro, and (d) the deficient patients fail to recruit PMN to sites of infection.

CD11b/CD18 has been previously described as a receptor for the third component C3bi [Wright et al., Proc. Natl. Acad. Sci. USA, 80, 5699-5703 (1983)]. It appears that a single receptor functions in two capacities: mediation of the binding of polymorphonuclear leukocytes (PMN) to endothelial cells; and to C3bi-coated cells.

Previous studies have also determined the amino acid sequence in C3bi recognized by CD11b/CD18 [Wright et al., Proc. Natl. Acad. Sci. USA, 84, 1965-1968 (1987)]. CD11b/CD18 binds to a sequence in C3bi that contains the sequence Lys-Tyr-Arg-Gly-Asp (human C3bi) or Lys-Tyr-Leu-Gly-Asp (murine C3bi). This was not completely unexpected since receptors related to CD11b/CD18 such as the fibronectin receptor recognize proteins that have the sequence Arg-Gly-Asp as part of the recognition site [Wright et al., J. Exp. Med., 162, 762-767 (1985); Ruoslahti et al. Cell, 44, 517-518 (1986)]. An important distinction exists between CD11b/CD18 and other receptors, such as the fibronectin receptor, which recognize Arg-Gly-Asp. Arg-Gly-Asp, in itself, is not recognized by CD11b/CD18. Thus, the identity of the flanking amino acids are critical to recognition by CD11b/CD18, and the Arg may be substituted by an uncharged amino acid if suitable flanking amino acids are present.

It is thus an object of the present invention to provide a method of inhibiting the binding of polymorphonuclear leukocytes (PMN) to endothelium and to fibrinogen using certain peptides and compositions containing said peptides.

It is a further object of the invention to provide a method of treating disease states wherein the inhibition of the binding of polymorphonuclear leukocytes (PMN) to endothelium and to fibrinogen will prevent or obviate the tissue damage which would result from such binding by administering to a patient in need of such therapy, an inhibitory peptide or a composition containing said peptide.

It is still a further object of the present invention to combine the inhibitory peptides of the present invention with carriers such as human serum albumin or high molecular weight dextran to prolong their presence in the bloodstream and thus enhance their therapeutic effects.

### SUMMARY OF THE INVENTION

The present invention relates to a method of and compositions for inhibiting the binding of polymorphonuclear leukocytes (PMN) to endothelium and to fibrinogen which comprises administration to a patient in need of such therapy an inhibitory amount of a peptide of the formula

$H_2N$-(Ch) - $X_1$-$X_2$-$X_3$-$B_1$-$X_4$-$B_2$-Gly-Asp-(Cx)-H

wherein (Ch) and (Cx) are each independently a chain of 0-8 naturally occurring amino acids and $B_1$, $B_2$, $X_1$, $X_2$, $X_3$ and $X_4$ are independently selected from a group consisting of naturally occurring amino acids.

### DESCRIPTION OF THE FIGURES

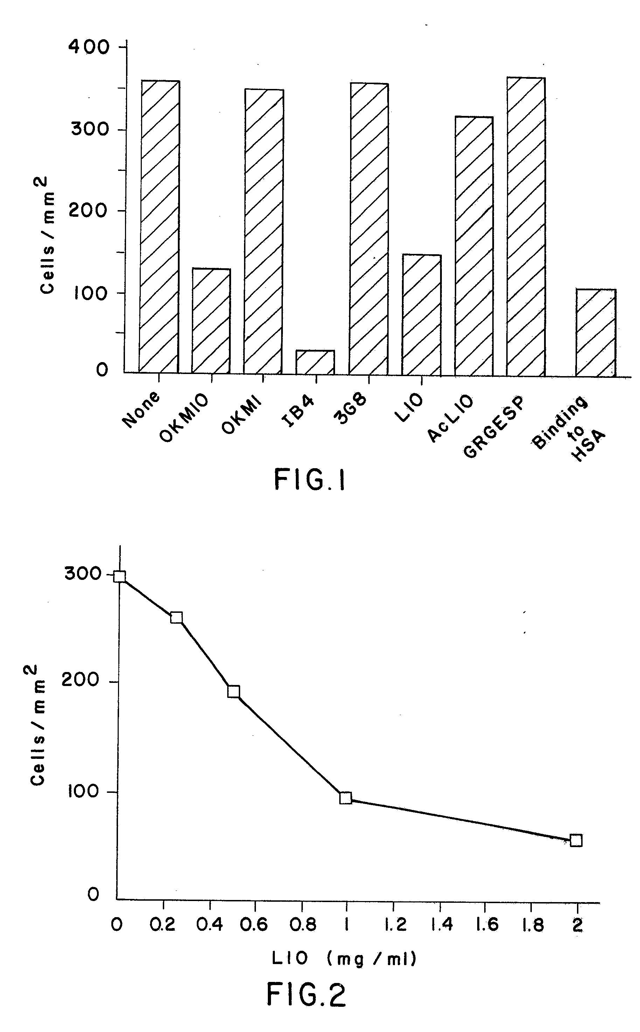

Figure 1 illustrates the inhibition of the binding of stimulated PMN to surfaces coated with fibrinogen. Monoclonal antibodies against CD11b/CD18 (OKM10 and IB4) reduce binding to the levels observed for attachment to surfaces coated with the control substance, human serum albumin, thus demonstrating the role of CD11b/CD18 in the interaction of PMN with fibrinogen. The synthetic peptide Leu-Gly-Gly-Ala-Lys-Gln-Ala-Gly-Asp-Val (L10) at 2 mg/ml also strongly inhibits binding to fibrinogen. The specificity of inhibition is shown by the absence of inhibition observed when the Lys residue of that peptide is modified by acetylation as in the peptide AcL10.

Figure 2 shows the dose dependence of the inhibition of the binding of PMN to fibrinogen by L10.

Figure 3 shows the dose dependence of the inhibition of the binding of PMN to endothelial cells by
Arg-Leu-Leu-Pro-Gly-Gly-Leu-Gln-Gln-Gly-Arg-Gly-Asp-Ala-Val-Gly-Pro-Glu-Arg-Gly-Cys  (Pep63).

Figure 4 shows the dose dependence of the inhibition of the binding of PMN to endothelial cells by
Gly-Gln-Gln-His-His-Leu-Gly-Gly-Ala-Lys-Gln-Ala-Gly-Asp-Val  (G15).

## DETAILED DESCRIPTION OF THE INVENTION

The present inventions relates to a method of and compositions for inhibiting the binding of polymorphonuclear leukocytes (PMN) to endothelium and to fibrinogen which comprises administration to a patient in need of such therapy an inhibitory amount of a peptide of the formula

$H_2N$-(Ch)-$X_1$-$X_2$-$X_3$-$B_1$-$X_4$-$B_2$-Gly-Asp-(Cx)-H    (I)

wherein Ch and Cx are each independently a chain of 0-8 amino acids and $B_1$, $B_2$, $X_1$, $X_2$, $X_3$ and $X_4$ are independently selected from a group consisting of amino acids with the proviso that the resulting peptide must contain at least ten amino acids.

The amino acids referred to above are the naturally occurring amino acids in either their L, D or DL stereochemical configuration. Throughout this disclosure, they are conveniently referred to by their conventional IUPAC three-letter abbreviations.

Of the peptides of formula I, certain combinations of the variables are preferred. Thus, preferred peptides of formula I are those wherein $X_1$ is glycine (Gly) and $B_1$ is lysine (Lys) and are thus represented by the formula

$H_2N$-(Ch)-Gly-$X_2$-$X_3$-Lys-$X_4$-$B_2$-Gly-Asp-(Cx)-H

wherein Ch and Cx are each independently a chain of 0-8 amino acids and $B_2$, $X_2$, $X_3$ and $X_4$ are each independently selected from the group consisting of amino acids.

In addition, one group of preferred peptides of formula I is that wherein the Ch chain of amino acids contains 0-5 amino acids which are selected from the group consisting of basic amino acids, i.e., lysine (Lys), arginine (Arg) and histidine (His).

In addition, a group of preferred peptides are those of formula I wherein $B_2$ is preferably selected from the group consisting of basic amino acids, i.e. lysine (Lys), arginine (Arg) and histidine (His).

A further preferred group of peptides of formula I utilizable in the present invention is that wherein one or more of the $X_1$, $X_2$, $X_3$ and $X_4$ amino acids are glycine (Gly).

Another group of highly preferred peptides of formula I utilizable in the present invention contains the subset of amino acids wherein
$B_1$-$X_4$-$B_2$-Gly-Asp-(Cx) is the -Lys-Gln-Ala-Gly-Asp-Val- group while another contains the subset of amino acids wherein
$X_4$-$B_2$-Gly-Asp-(Cx) is  the - Gly-Arg-Gly-Asp-Ala-Val-($C_{x-1}$) group.

The term $C_{x-1}$ denotes a chain of 1-7 amino acids independently selected from the group consisting of amino acids.

Thus, preferred peptides for use in the methods and compositions of the present invention are those of the formulae:
$H_2N$-Leu-Gly-Gly-Ala-Lys-Gln-Ala-Gly-Asp-Val-H;
$H_2N$-Gly-Gln-Gln-His-His-Leu-Gly-Gly-Ala-Lys-Gln-Ala-Gly-Asp-Val-H; and
$H_2N$-Arg-Leu-Leu-Pro-Gly-Gly-Leu-Gln-Gln-Gly-Arg-Gly-Asp-Ala-Val-Gly-Pro-Glu-Arg-Gly-Cys-H

Certain of the peptides of the present invention are novel entities. These peptides either contain one or more D-configuration amino acids or contain a sequence derived from the naturally occurring gamma chain of the fibrinogen molecule, i.e., one or more of the amino acids of the sequence of the peptide is different from the sequence of amino acids of the gamma chain of human fibrinogen. Other of the peptides utilized in the present invention are novel although they contain sequences that occur in naturally occurring proteins since they have never before been produced as entities apart from the naturally occurring proteins. For instance, the peptide Pep63 of the formula
Arg-Leu-Leu-Pro-Gly-Gly-Leu-Gln-Gln-Gly-Arg-Gly-Asp-Ala-Val-Gly-Pro-Glu-Arg-Gly-Cys
is derived from a surface glycoprotein gp 63 of Leishmania promastigotes. However, Pep63 itself in a purified form has heretofore been unknown and unavailable as a therapeutic agent.

The peptides utilizable in the methods and compositions of the present invention exhibit their utility by inhibiting the binding of PMN to endothelium and also by inhibiting the binding of PMN to the clotting protein fibrinogen. Thus, when peptides of formula I are incubated with human PMN, they inhibit the binding of PMN to both endothelium and fibrinogen as determined by standardized assays. These peptides of formula I can be utilized to treat a patient in need of therapy due to the damage which would result from the binding of PMN to endothelium and/or to fibrinogen. Since the binding of PMN to endothelium and to fibrinogen results in tissue destruction during or after strokes, heart disease, meningitis, pneumococcal pneumonia, otitis media, adult respiratory distress syndrome, gout, asthma, psoriasis, Reiter's syndrome, leukoplastic vasculitis, Sweet's syndrome, pustular vasculitis, erythema nodosum, pyoderma gangrenosum, obstructive trauma, acute rejection of transplanted organs, emphysema and septic shock, patients afflicted with any of these disease states are candidates for the therapeutic methods and compositions utilizing the peptides of formula I.

The peptides are preferably prepared using solid phase synthesis, such as that described by Merrifield [J. Am. Chem. Soc., 85, 2149 (1964)], although other equivalent chemical syntheses known in the art can also be used, such as the syntheses of Houghten [Proc. Natl. Acad. Sci., 82, 5132 (1985)]. Solid-phase synthesis is commenced from the C-terminus of the peptide by coupling a protected amino acid to a suitable resin, as generally set forth in U.S. Patent No. 4,244,946, issued January 21, 1982 to Rivier et al. Examples of syntheses of this general type are set forth in U.S. Patent Nos. 4,305,872 and 4,316,891. Discussion of the solid phase synthesis of a 41-residue polypeptide is set forth in Science, 213, 1394-1397 (September, 1981) in an article by Vale et al., which refers to a more detailed discussion of the synthesis, which appears in an article by Marke et al. in J. Am. Chem. Soc., 103, 3178 (1981).

In synthesizing the polypeptide, valine or serine having its alpha-amino group suitably protected (and, with serine, having its hydroxymethyl side chain protected) is coupled to a chloromethylated polystyrene resin or the like. After removal of the alpha-amino protecting group, as by using trifluoroacetic acid in methylene chloride, the next step in the synthesis is ready to proceed. Other standard cleaving reagents and conditions for the removal of specific amino protecting groups may be used, as described in the open literature.

The remaining alpha-amino- and side-chain-protected amino acids are then coupled stepwise in the desired order to obtain an intermediate compound connected to the resin. As an alternative to adding each amino acid separately in the synthesis, some of them may be coupled to one another prior to the addition to the growing solid-phase chain. The selection of the appropriate coupling reagents is within the skill of the art.

Common to chemical syntheses of peptides is the protection of the labile side-chain groups of the various amino acid moieties with suitable protecting groups at that site until the group is ultimately removed after the chain has been completely assembled. Also common is the protection of the alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in the desired sequence in the peptide chain with various of these residues having side-chain protecting groups. These protecting groups are then commonly removed substantially at the same time so as to produce the desired resultant product following purification.

Peptides composed of repeating units of the peptide of formula (I) above are made by the same procedure.

After the desired amino acid sequence has been completed, the intermediate peptide is removed from the resin support by treatment with a reagent, such as liquid HF, which not only cleaves the peptide from the resin, but also cleaves all the remaining side-chain-protecting groups. The polypeptide can then be purified by gel permeation followed by semipreparative HPLC, as described in Rivier et al. [Peptides: Structure and Biological Function, 125-128 (1979)]. A purity of at least 93% or higher (based upon all peptides present) is reasonably obtainable and is preferred for clinical testing and/or use. Purity of 98% is practical; however, for certain in vitro applications, lower purity may be acceptable. Accordingly, the polypeptide is considered useful when it is in substantially pure form which, for purposes of this Application, means at least about 50 weight percent, based upon all peptides present.

The peptides of the present invention can be formulated into pharmaceutical compositions for therapeutic, diagnostic or other uses. To prepare them for intravenous administration, the compositions are dissolved in water containing physiologically compatible substances such as sodium chloride (e.g., 0.1 -2.0 M), glycine, and the like and having buffered pH compatible with physiological conditions. The amount to administer will depend on the activity of the particular compound administered, which can readily be determined by those of ordinary skill in the art.

Additionally, in order to prolong the therapeutic effect of the peptides of the present invention, the peptides of the present invention can be conjugated with a carrier molecule such as human serum albumin or high molecular weight dextran. This results in a larger molecule which is not cleared so quickly from the bloodstream of the patient. After conjugation, the peptide can then be formulated into suitable compositions for administration to the patient in need of such therapy.

The following examples illustrate the preparations and methods of the invention. In the following examples, all peptides are synthesized using standard automated methods (Applied Biosystems), and are purified by high pressure liquid chromatography using standard methods. After lyophilization, peptides are weighed, dissolved in physiological saline, and the pH is adjusted to neutrality by the addition of sodium hydroxide.

## EXAMPLE 1

This example illustrates that the method of the invention can be used to inhibit binding of stimulated PMN to fibrinogen-coated surfaces. Figure 1 illustrates the level of inhibition of binding by incubation with monoclonal antibodies against CD11b/CD18 and with the peptide L10, and Figure 2 illustrates the dose dependence of this inhibition.

Details of the experiments are as follows. Purified human PMN were stimulated for 15 min. with 300 ng/ml of phorbol dibutyrate. This stimulation is necessary to enable PMN to bind, and mimics the stimulation of PMN at sites of inflammation. Culture wells were coated with fibrinogen or human serum albumin by incubating 1 mg/ml of these proteins with the culture surfaces for 1 hr. at 20°C. The PMN were then washed and added to culture wells coated with fibrinogen in the presence of 10µg/ml of various monoclonal antibodies or 2 mg/ml of synthetic peptides. PMN were allowed to settle onto the surface for 20 min. at 0°C, then were warmed to 37°C for 30 min. The surfaces were then washed and the number of cells adherent were counted by phase contrast microscopy.

Figure 1 illustrates that PMN bind poorly to surfaces coated with human serum albumin, but bind strongly to surfaces coated with fibrinogen. The binding of PMN to fibrinogen is mediated by CD11b/CD18 since the monoclonal antibodies OKM10 (against CD11b) and IB4 (against CD18) block binding, while monoclonal antibody against an irrelevant protein (3G8, against the Fc receptor) has no effect. These data further show that the peptide L10
Leu-Gly-Gly-Ala-Lys-Gln-Ala-Gly-Asp-Val
strongly inhibits binding of PMN to fibrinogen . The specificity of the inhibition is indicated by the observation that the peptide Gly-Arg-Gly-Glu-Ser-Pro does not inhibit binding to fibrinogen, and acetylation of the Lys group in L10 as in the peptide AcL10, causes loss of the inhibitory capacity. (Acetylation of the Lys group was performed by incubating L10 with acetic anhydride as described [J. Biol. Chem., 262. 947-950 (1987)].

Figure 2 illustrates the dose dependence of the inhibition of binding of PMN to fibrinogen by L10.

Figures 1 and 2 thus demonstrate that the attachment of PMN to fibrinogen may be inhibited by the method of the invention.

## EXAMPLE 2

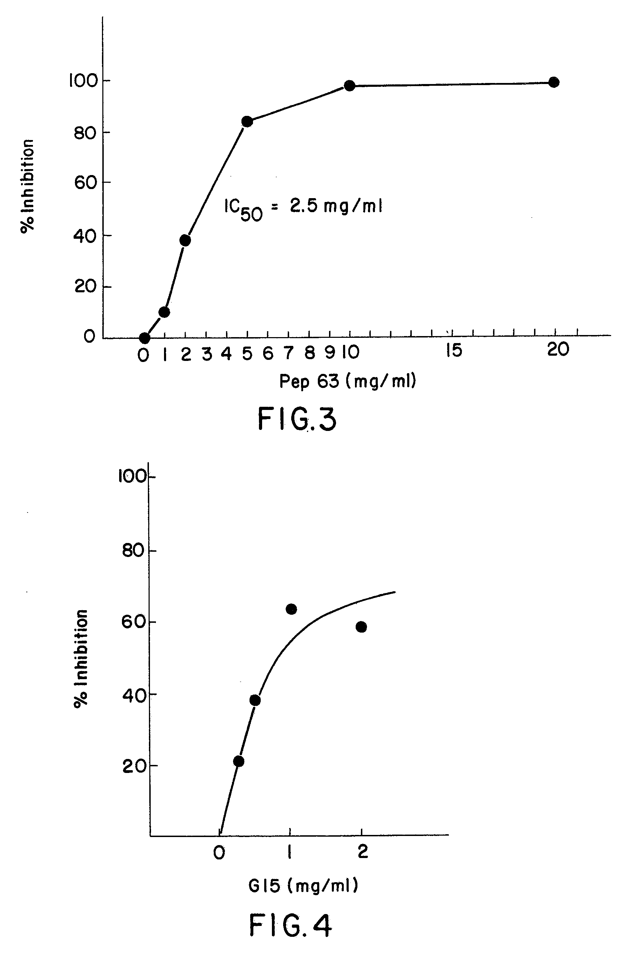

This example illustrates that the method of the invention can be used to inhibit the binding of stimulated PMN to human endothelial cells. Figure 3 describes the dose dependence of inhibition caused by the peptide Pep63, and Figure 4 describes the dose dependence of inhibition caused by the peptide G15.

The experiments are conducted as follows. Purified human PMN were stimulated for 15 min. with 300 ng/ml of phorbol dibutyrate. This stimulation is necessary to enable PMN to bind, and mimics the stimulation of PMN at sites of inflammation. Endothelial cells were isolated from human umbilical cords by standard methods [Pawlowski et al., Proc. Natl. Aca. Sci., 82, 8202 (1985)] and were cultivated on fibronectin-coated tissue culture surfaces to confluence. The endothelial cells exhibited the morphological features of native endothelium. PMN were washed and combined with the indicated doses of
Arg-Leu-Leu-Pro-Gly-Gly-Leu-Gln-Gln-Gly-Arg-Gly-Asp-Ala-Val-Gly-Pro-Glu-Arg-Gly-Cys   (Pep63)
in Figure 3, or with the indicated doses of
Gly-Gln-Gln-His-His-Leu-Gly-Gly-Ala-Lys-Gln-Ala-Gly-Asp-Val   (G15)
in Figure 4. The cells were then added to the endothelium and incubated at 37°C for 15 min. Unattached PMN were then washed away and the binding was assessed by microscopy.

Figures 3 and 4 indicate that the method of the invention is effective in blocking the attachment of stimulated PMN to endothelium.

This invention may be embodied in other forms or carried out in other ways without departing from the spirit or essential characteristics thereof. The present disclosure is therefore to be considered as in all respects illustrative and not restrictive, the scope of the invention being indicated by the appended Claims, and all changes which come within the meaning and range of equivalency are intended to be embraced therein.

## Claims

1. An agent for inhibiting the binding of polymorphonuclear leukocytes (PMN) to endothelium and to fibrinogen which comprises a peptide of the formula
$H_2N-(Ch)-X_1-X_2-X_3-B_1-X_4-B_2-Gly-Asp-(Cx)-H$   (I)
wherein Ch and Cx are each independently a chain of 0-8 amino acids and $B_1$, $B_2$, $X_1$, $X_2$, $X_3$ and $X_4$ are independently selected from a group consisting of amino acids with the proviso that the peptide contains at least ten amino acids.

2. The agent of Claim 1 wherein $X_1$ is glycine (Gly) and $B_1$ is lysine (Lys).

3. The agent of Claim 1 wherein $B_2$ is selected from the group consisting of lysine (Lys), arginine (Arg) and histidine (His).

4. The agent of Claim 1 wherein the Ch amino acids are selected from the group consisting of lysine (Lys), arginine (Arg) and histidine (His).

5. The agent of Claim 1 wherein one or more of the $X_1$, $X_2$, $X_3$ and $X_4$ amino acids are glycine (Gly).

6. The agent of Claim 1 wherein the peptide is $H_2N$-Leu-Gly-Gly-Ala-Lys-Gln-Ala-Gly-Asp-Val-H.

7. The agent of Claim 1 wherein the peptide is $H_2N$-Gly-Gln-Gln-His-His-Leu-Gly-Gly-Ala-Lys-Gln-Ala-Gly-Asp-Val-H.

8. The agent of Claim 1 wherein the peptide has the formula
$H_2N$-Arg-Leu-Leu-Pro-Gly-Gly-Leu-Gln-Gln-Gly-Arg- Gly-Asp-Ala-Val-Gly-Pro-Glu-Arg-Gly-Cys-H.

9. The agent of Claim 1 wherein the peptide contains the subset of amino acids
-Lys-Gln-Ala-Gly-Asp-Val- .

10. The agent of Claim 1 wherein the peptide contains the subset of amino acids
- Gly-Arg-Gly-Asp-Ala-Val-

11. The agent of Claim 1 wherein the peptide is grafted to a carrier molecule.

12. The agent of Claim 2 wherein the peptide is grafted to a carrier molecule.

13. The agent of Claim 1 wherein the carrier molecule is human serum albumin.

14. The agent of Claim 2 wherein the said carrier molecule is human serum albumin.

15. The agent of Claim 1 wherein the said carrier molecule is high molecular weight dextran.

16. The agent of Claim 2 wherein the said carrier molecule is high molecular weight dextran.

17. A pharmaceutical composition for inhibiting the binding of polymorphonuclear leukocytes (PMN) to endothelium and to fibrinigen comprising the agent of any of Claims 1-17 and a pharaceutically acceptable carrier.

18. The use of an agent comprising peptide of the formula

$\text{H}_2\text{N-(Ch)-X}_1\text{-X}_2\text{-X}_3\text{-B}_1\text{-X}_4\text{-B}_2\text{-Gly-Asp-}$
$\text{(Cx)-H}$     (I)

wherein Ch and Cx are each independently a chain of 0-8 amino acids and $\text{B}_1$, $\text{B}_2$, $\text{X}_1$, $\text{X}_2$, $\text{X}_3$ and $\text{X}_4$ are independently selected from the group consisting of amino acids, with the proviso that the peptide contains at least ten amino acids; for the manufacture of a composition useful for inhibiting the binding of polymorphonuclear leukocytes (PMN) to endothelium and to fibrinogen in a patient in need of such therapy.

19. The use of Claim 18 wherein $\text{X}_1$ is glycine (Gly) and $\text{B}_1$ is lysine (Lys).

20. The use of Claim 18 wherein $\text{B}_2$ is selected from the group consisting of lysine (Lys), arginine (Arg) and histidine (His).

21. The use of Claim 18 wherein the Ch amino acids are selected from the group consisting of lysine (Lys), arginine (Arg) and histidine (His).

22. The use of Claim 18 wherein one or more of the
$\text{X}_1$, $\text{X}_2$, $\text{X}_3$ and $\text{X}_4$ amino acids are glycine (Gly).

23. The use of Claim 18 wherein the peptide is
$\text{H}_2\text{N-Leu-Gly-Gly-Ala-Lys-Gln-Ala-Gly-Asp-Val-H.}$

24. The use of Claim 18 wherein the peptide is
$\text{H}_2\text{N-Gly-Gln-Gln-His-His-Leu-Gly-Gly-Ala-Lys-Gln-Ala-Gly-Asp-Val-H.}$

25. The use of Claim 18 wherein the peptide has the formula
$\text{H}_2\text{N-Arg-Leu-Leu-Pro-Gly-Gly-Leu-Gln-Gln-Gly-Arg-Gly-Asp-Ala-Val-Gly-Pro-Glu-Arg-Gly-Cys-H.}$

26. The use of Claim 18 wherein the peptide contains the subset of amino acids
-Lys-Gln-Ala-Gly-Asp-Val- .

27. The use of Claim 18 wherein the peptide contains the subset of amino acids
-Gly-Arg-Gly-Asp-Ala-Val- .

28. The use of Claim 18 wherein the peptide is grafted to a carrier molecule.

29. The use of Claim 19 wherein the peptide is grafted to a carrier molecule.

30. The use of Claim 18 wherein the carrier molecule is human serum albumin.

31. The use of Claim 19 wherein the said carrier molecule is human serum albumin.

32. The use of Claim 18 wherein the said carrier molecule is high molecular weight dextran.

33. The use of Claim 19 wherein the said carrier molecule is high molecular weight dextran.

34. A purified peptide of the formula
Arg-Leu-Leu-Pro-Gly-Gly-Leu-Gln-Gln-Gly-Arg-Gly-Asp-Ala-Val-Gly-Pro-Glu-Arg-Gly-Cys     (Pep63).

FIG.1

FIG.2

FIG.3

FIG.4